(19) Europäisches Patentamt
European Patent Office
Office européen des brevets

(11) **EP 4 097 076 B1**

(12) **EUROPEAN PATENT SPECIFICATION**

(45) Date of publication and mention of the grant of the patent:
**01.05.2024 Bulletin 2024/18**

(21) Application number: **21706751.1**

(22) Date of filing: **21.01.2021**

(51) International Patent Classification (IPC):
***C07C 229/12*** (2006.01) ***C11D 1/46*** (2006.01)
***A61K 8/44*** (2006.01) ***A61Q 5/12*** (2006.01)
***A61Q 5/02*** (2006.01) ***A01N 25/30*** (2006.01)
***C09D 5/02*** (2006.01) ***C09D 7/63*** (2018.01)

(52) Cooperative Patent Classification (CPC):
**C07C 229/12; A01N 25/30; A61K 8/44; A61Q 5/02; A61Q 5/12; C09D 5/021; C09D 7/63; C11D 1/46;** C08K 5/175

(86) International application number:
**PCT/US2021/014440**

(87) International publication number:
**WO 2021/154582 (05.08.2021 Gazette 2021/31)**

(54) **AMINO ACID SURFACTANTS**

AMINOSÄURETENSIDE

TENSIOACTIFS D'ACIDES AMINÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.01.2020 US 202062967170 P**

(43) Date of publication of application:
**07.12.2022 Bulletin 2022/49**

(73) Proprietor: **AdvanSix Resins & Chemicals LLC**
**Parsippany, New Jersey 07054 (US)**

(72) Inventors:
- **ASIRVATHAM, Edward**
  **Parsippany, New Jersey 07054 (US)**
- **HONCIUC, Andrei**
  **Parsippany, New Jersey 07054 (US)**
- **MIHALI, Voichita**
  **Parsippany, New Jersey 07054 (US)**

(74) Representative: **Crooks, Elizabeth Caroline et al**
**Kilburn & Strode LLP**
**Lacon London**
**84 Theobalds Road**
**London WC1X 8NL (GB)**

(56) References cited:
**EP-A2- 0 826 661**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 097 076 B1

## Description

CROSS-REFERENCE TO RELATED APPLICATION

This application claims priority to U.S. Provisional Application No. 62/967,170, filed January 29, 2020.

FIELD

The present disclosure pertains to derivatives of amino acids and methods for their synthesis, wherein the amino acid derivatives have surface-active properties.

BACKGROUND

Surfactants (molecules with surface-active properties) are an important class of molecules with highly sought-after characteristics. Surfactants may be uncharged, zwitterionic, cationic, or anionic. Often, these compounds are amphiphilic molecules with a water-insoluble hydrophobic "tail" group and a watersoluble hydrophilic "head" group. These compounds may adsorb at an interface, such as an interface between two liquids, a liquid and a gas, or a liquid and a solid. In the case of an interface between water and oil, the hydrophilic head group extends into the water, while the hydrophobic tail extends into the oil. When added to water, the hydrophilic head group extends into the water, while the hydrophobic tail extends into the air. The presence of the surfactant disrupts the intermolecular interaction between water molecules, replacing it with weaker interactions between water molecules and the surfactant. This results in lowered surface tension and can also serve to stabilize the interface.

At sufficiently high concentrations, surfactants may form aggregates to limit the exposure of the hydrophobic tail to the polar solvent. One such aggregate is a micelle, in which the molecules are arranged in a sphere with the hydrophobic tails inside the sphere and the hydrophilic heads on the outside to interact with a polar solvent. The effect that a given compound has on surface tension and the concentration at which it forms micelles may serve as defining characteristics for a surfactant.

Surfactants are widely used in commercial applications in formulations ranging from detergents to hair care products to cosmetics. Compounds with surface-active properties are used as soaps, detergents, lubricants, wetting agents, foaming agents, and spreading agents, among others. Thus, there is an ongoing need to identify and synthesize such compounds. EP 0 826 661 A2 discloses compounds having the formula R1 R2R3N$^{\oplus}$-Y-COOR A$^{\ominus}$ which are useful in the treatment of hair.

However, solely from its structure, it may be difficult to predict whether a given compound would have surface-active properties, let alone other important characteristics such as interfacial adsorption dynamics, minimum surface tension achievable, and/or ability to wet hydrophobic and/or oleophobic surfaces, which are also integral to whether the compound would become a useful surfactant. Certain amino acids and their derivatives, for example, are desirable as building blocks for surfactants, but the selection of which amino acids to use is far from intuitive. Synthesis of such compounds adds another layer of difficulty due to the differences of solubilities attributable to different elements and moieties present in the same molecules. There remains a need for high-efficacy surfactants that can be readily synthesized at commercial scale via straightforward routes.

SUMMARY

The present disclosure provides derivatives of amino acids that have surface-active properties. The amino acids may be naturally occurring or synthetic amino acids, or they may be obtained via ring-opening reactions of molecules such as lactams, for example caprolactam. The amino acids may be functionalized to form compounds with surface-active properties. Characteristically, these compounds may have low critical micelle concentrations (CMC) and/or the ability to reduce the surface tension of a liquid.

The present disclosure provides compounds of Formula I, below, also referred to herein as the surfactant:

R2 R1 O N⊕ R3 n O m X⊖

Formula I

wherein $R^1$, $R^2$, and $R^3$ are independently chosen from hydrogen and $C_1$-$C_6$ alkyl, namely $C_1$, $C_2$, $C_3$, $C_4$, $C_5$, or $C_6$; n is an integer from 2 to 5, namely 2, 3, 4, or 5; m is an integer from 9 to 20, namely 9, 10, 11, 12, 13, 14, 15, 16, 17, 18, 19, or 20; and X is an anion that may be selected from the group consisting of chloride, bromide, iodide and hydroxide.

The present invention provides compounds of Formula II, below, also referred to herein as the surfactant:

Formula II

wherein X is an anion that may be selected from the group consisting of chloride, bromide, iodide, and hydroxide.

One specific compound provided by the present invention is 6-(dodecyloxy)-N,N-dimethyl-6-oxohexan-1-aminium chloride, having the following formula:

The above mentioned and other features of the disclosure, and the manner of attaining them, will become more apparent and will be better understood by reference to the following description of embodiments taken in conjunction with the accompanying drawings.

## BRIEF DESCRIPTION OF THE DRAWINGS

Fig. 1 shows a plot of surface tension versus concentration measured at pH = 7 as described in Example 2, wherein the Y axis depicts the surface tension ($\gamma$) in millinewtons per meter (mN/m) and the X axis depicts the concentration (c) in millimoles (mM).

Fig. 2 shows a plot of dynamic surface tension as change in surface tension versus time as described in Example 3, wherein the Y axis depicts the surface tension in millinewtons per meter (mN/m) and the X axis depicts the surface age in milliseconds (ms).

## DETAILED DESCRIPTION

As used herein, the phrase "within any range defined between any two of the foregoing values" literally means that any range may be selected from any two of the values listed prior to such phrase regardless of whether the values are in the lower part of the listing or in the higher part of the listing. For example, a pair of values may be selected from two lower values, two higher values, or a lower value and a higher value.

As used herein, the word "alkyl" means any saturated carbon chain, which may be a straight or branched chain.

As used herein, the phrase "surface-active" means that the associated compound is able to lower the surface tension of the medium in which it is dissolved, and/or the interfacial tension with other phases, and, accordingly, may be adsorbed at the liquid/vapor and/or other interfaces. The term "surfactant" may be applied to such a compound.

With respect terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error or minor adjustments made to optimize performance, for example. In the event it is determined that individuals having ordinary skill in the relevant arts would not readily ascertain values for such reasonably small differences, the terms "about" and "approximately" can be understood to mean plus or minus 10% of the stated value.

The present disclosure provides derivatives of amino acids. The amino acids may be naturally occurring or synthetic, or they may be obtained from ring-opening reactions of lactams, such as caprolactam. The compounds of the present disclosure have been shown to have surface-active properties, and may be used as surfactants and wetting

agents, for example. In particular, the present disclosure provides compounds of Formula I, shown below:

Formula I

wherein $R^1$, $R^2$, and $R^3$ are independently chosen from hydrogen and $C_1$-$C_6$ alkyl, namely $C_1$, $C_2$, $C_3$, $C_4$, $C_5$, or $C_6$; n is an integer from 2 to 5, namely 2, 3, 4, or 5; m is an integer from 9 to 20, namely 9, 10, 11, 12, 13, 14, 15, 16, 17, 18, 19, or 20; and X is an anion that may be selected from the group consisting of chloride, bromide, iodide, and hydroxide.

The present invention provides for compounds of Formula II, shown below:

Formula II

wherein X is an anion that may be selected from the group consisting of chloride, bromide, iodide, and hydroxide.

One specific compound provided by the present invention is 6-(dodecyloxy)-N,N-dimethyl-6-oxohexan-1-aminium chloride, having the following formula:

These compounds may be synthesized by various methods. One such method includes opening a lactam to yield an amino acid having an N-terminus and reacting the N-terminus of the amino acid with an alkylating agent to yield a tertiary amine. The resulting tertiary amine may then react with an alcohol under acidic conditions to provide an amino acid ester having an N-terminus. The amino acid ester N-terminus may then react with an acid to yield a quaternary amine salt.

The amino acid may be naturally occurring or synthetic or may be derived from a ring opening reaction of a lactam, such as propiolactam, butyrolactam, valerolactam, and caprolactam, for example. The ring-opening reaction may be either an acid or alkali catalyzed reaction, and an example of an acid catalyzed reaction is shown below in Scheme 1.

SCHEME 1

$H_2SO_4$ / $H_2O$

The amino acid may have as few as 2 or as many as 5, namely 2, 3, 4, or 5, carbons between the N- and C-termini. The alkyl chain may be branched or straight. The alkyl chain may be interrupted with nitrogen, oxygen, or sulfur. The alkyl chain may be further substituted with one or more substituents selected from the group consisting of hydroxyl, amino, amido, sulfonyl, sulfonate, carboxyl, and carboxylate. The N-terminal nitrogen may be acylated or alkylated with one or more alkyl groups. For example, the amino acid may be 6-(dimethylamino)hexanoic acid.

The derivative of the amino acid may be synthesized as shown below in Scheme 2. As shown, 6-aminohexanoic acid is treated with formaldehyde in formic acid at reflux to give 6-(dimethylamino)hexanoic acid. The free carboxylic acid is then treated with an alcohol, such as dodecanol, in the presence of *p*-toluene sulfonic acid (PTSA) in toluene to

give the corresponding ester, dodecyl 6-(dimethylamino)hexanoate. The N-terminus is then alkylated with methyl iodide in the presence of sodium carbonate.

SCHEME 2


HO
NH2
H
H
H
OH
reflux
HO
N
O
OH
+
OH
10
PTSA
toluene
N
O
O
10
HCl
H
Cl
N
O
O


The compounds of the present disclosure demonstrate surface-active properties. These properties may be measured and described by various methods. One method by which surfactants may be described is by the molecule's critical micelle concentration (CMC). CMC may be defined as the concentration of a surfactant at which micelles form, and above which all additional surfactant is incorporated into micelles.

As surfactant concentration increases, surface tension decreases. Once the surface is completely overlaid with surfactant molecules, micelles begin to form. This point represents the CMC, as well as the minimum surface tension. Further addition of surfactant will not further affect the surface tension. CMC may therefore be measured by observing the change in surface tension as a function of surfactant concentration. One such method for measuring this value is the Wilhemy plate method. A Wilhelmy plate is usually a thin iridium-platinum plate attached to a balance by a wire and placed perpendicularly to the air-liquid interface. The balance is used to measure the force exerted on the plate by wetting. This value is then used to calculate the surface tension ($\gamma$) according to Equation 1:

$$\text{Equation 1: } \gamma = F/l \cos \theta$$

wherein l is equal to the wetted perimeter (2w + 2d, in which w and d are the plate thickness and width, respectively) and cos 8, the contact angle between the liquid and the plate, is assumed to be 0 in the absence of an extant literature value.

Another parameter used to assess the performance of surfactants is dynamic surface tension. The dynamic surface tension is the value of the surface tension for a particular surface or interface age. In the case of liquids with added surfactants, this can differ from the equilibrium value. Immediately after a surface is produced, the surface tension is equal to that of the pure liquid. As described above, surfactants reduce surface tension; therefore, the surface tension drops until an equilibrium value is reached. The time required for equilibrium to be reached depends on the diffusion rate and the adsorption rate of the surfactant.

One method by which dynamic surface tension is measured relies upon a bubble pressure tensiometer. This device measures the maximum internal pressure of a gas bubble that is formed in a liquid by means of a capillary. The measured value corresponds to the surface tension at a certain surface age, the time from the start of the bubble formation to the occurrence of the pressure maximum. The dependence of surface tension on surface age can be measured by varying the speed at which bubbles are produced.

Surface-active compounds may also be assessed by their wetting ability on solid substrates as measured by the contact angle. When a liquid droplet comes in contact with a solid surface in a third medium, such as air, a three-phase line forms among the liquid, the gas and the solid. The angle between the surface tension unit vector, acting at the three-phase line and tangent at the liquid droplet, and the surface is described as the contact angle. The contact angle (also known as wetting angle) is a measure of the wettability of a solid by a liquid. In the case of complete wetting, the liquid is completely spread over the solid and the contact angle is 0°. Wetting properties are typically measured for a given compound at the concentration of 1-100x CMC, however, it is not a property that is concentration-dependent therefore measurements of wetting properties can be measured at concentrations that are higher or lower.

In one method, an optical contact angle goniometer may be used to measure the contact angle. This device uses a digital camera and software to extract the contact angle by analyzing the contour shape of a sessile droplet of liquid on a surface.

Potential applications for the surface-active compounds of the present disclosure include formulations for use

as shampoos, hair conditioners, detergents, spot-free rinsing solutions, floor and carpet cleaners, cleaning agents for graffiti removal, wetting agents for crop protection, adjuvants for crop protection, and wetting agents for aerosol spray coatings.

It will be understood by one skilled in the art that small differences between compounds may lead to substantially different surfactant properties, such that different compounds may be used with different substrates, in different applications.

The following non-limiting embodiments are provided to demonstrate the different properties of the different surfactants.

The compounds are effective as surface-active agents, useful for wetting or foaming agents, dispersants, emulsifiers, and detergents, among other applications.

The compounds of the present disclosure may be useful in both the applications described above and some further special applications such as surface treatments, such as in personal hair care products, and can also be used to generate water repellant surfaces.

The amount of the compounds disclosed herein used in a formulation may be as low as about 0.001 wt.%, about 0.05 wt.%, about 0.1 wt.%, about 0.5 wt.%, about 1 wt.%, about 2 wt.%, or about 5 wt.%, or as high as about 8 wt.%, about 10 wt.%, about 15 wt.%, about 20 wt.%, or about 25 wt.%, or within any range defined between any two of the foregoing values.

## EXAMPLES

Nuclear magnetic resonance (NMR) spectroscopy was performed on a Bruker 500 MHz spectrometer. The critical micelle concentration (CMC) was determined by the Wilhelmy plate method at 23° C with a tensiometer (DCAT 11, DataPhysics Instruments GmbH) equipped with a Pt-Ir plate. Dynamic surface tension was determined with a bubble pressure tensiometer (Krüss BP100, Krüss GmbH), at 23° C. Contact angle was determined with the optical contact angle goniometer (OCA 15 Pro, DataPhysics GmbH) equipped with a digital camera.

### Example 1:

### Synthesis of 6-(dodecyloxy)-N,N-dimethyl-6-oxohexan-1-aminium chloride

6-(Dimethylamino)hexanoic acid (11.99 g, 75.36 mmol) was dissolved in toluene (50 mL) in a round bottom flask equipped with a Dean-Stark trap. Dodecanol (12.68 g, 75.36 mmol) and *p*-toluene sulfonic acid monohydrate (PTSA) (14.33 g, 75.36 mmol) were then added. The reaction was heated to reflux for 24 hours, until no further water was noted in the Dean-Stark trap. The solvent was removed under vacuum and the resultant solid was washed with hexanes. The solid was dissolved in dichloromethane (200 mL) and washed with saturated sodium carbonate to give dodecyl 6-(dimethylamino)hexanoate in 51% yield. $^1$H NMR (DMSO) δ 4.00 (t, *J* = 6.5 Hz, 2H), 2.27 (t, *J* = 7.3 Hz, 2H), 2.13-2.16 (m, 2H), 2.01 (s, 6H), 1.54 - 1.53 (m, 6H), 1.27-1.18 (m, 20H), 0.86 (t, 3H).

Dodecyl 6-(dimethylamino)hexanoate (100 mg, 0.305 mmol) was dissolved in water (10 mL). Concentrated hydrochloric acid (11.14 mg, 0.305 mmol) was added.

### Example 2:

### Determination of critical micelle concentration (CMC)

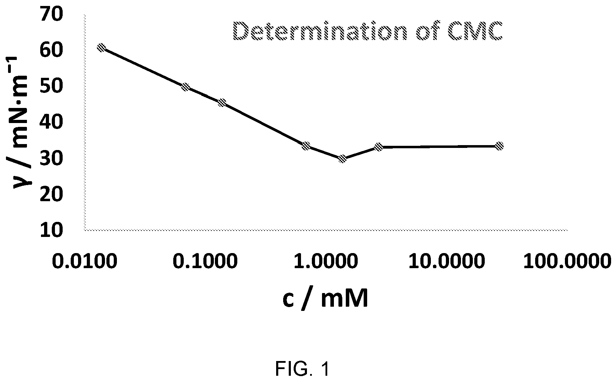

The critical micelle concentration (CMC) was tested. From the change in surface tension with concentration in water, the CMC was determined to be about 1.4 mmol. The plateau value of minimum surface tension that can be reached by this surfactant is about 30 mN/m, namely 30 mN/m ± 3 mN/m. Fig. 1 is a plot of these results, showing surface tension versus concentration. From the plot of the results, the surface tension at the CMC is equal to or less than about 30 mN/m. The plot further shows the surface tension to be equal to or less than 33 mN/m at a concentration of 2.7 mmol or greater.

### Example 3:

### Determination of dynamic surface tension

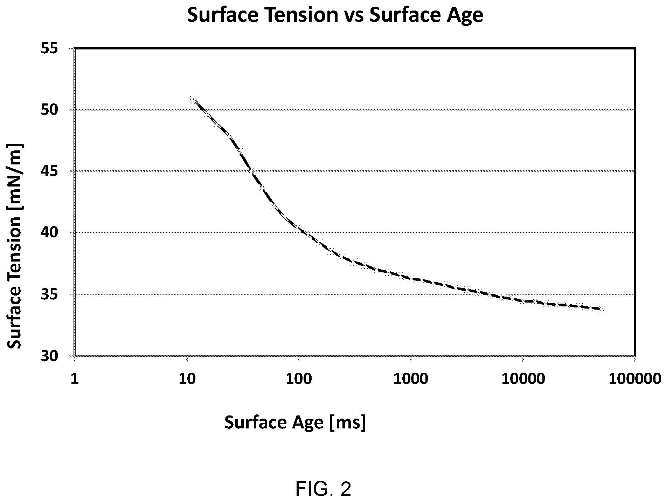

The dynamic surface tension was determined with a bubble pressure tensiometer which measures the change of surface tension of a freshly created air-water interface with time. Fig. 2 presents a plot of the surface tension versus time, showing that surface tension in the time interval between 1 and 100 ms drops rapidly from about 50 mN/m to about

40 mN/m. In the time interval from 100 to 50,000 ms, the surface tension drops slowly from 40 mN/m to about 34 mN/m, approaching asymptotically the saturation value of the surface tension at the CMC.

Example 4:

Determination of wetting properties

In addition to surface tension and surface dynamics, the wetting properties of the compound were tested on various surfaces. For example, hydrophobic substrates such as polyethylene-HD exhibit surface wetting with a contact angle of 42.5°. On oleophobic and hydrophobic substrates such as Teflon, the measured contact angle was much less than that of water, 66.6° (Table 1).

TABLE 1

| Substrate | CA of Surfactant (°) | Concentration | CA of water (°) |
|---|---|---|---|
| Teflon | 66.6 | 10x CMC | 119 |
| Polyethylene-HD | 42.5 | 10x CMC | 93.6 |
| Nylon | 15 | 10x CMC | 50 |
| Polyethylene terephthalate | 18.3 | 10x CMC | 65.3 |

Example 5:

Formulation for shampoo

In this Example, a formulation for use as a shampoo is provided. This formulation is useful in in providing hair with a smooth and silky feel. The components of the formulation are shown below in Table 2. Additionally, the formulation may include other natural oils and ingredients, as well as vitamins for consumer appeal, in an amount of less than 1 wt.%.

TABLE 2

| Component | Function | Weight % |
|---|---|---|
| Surfactant | Surfactant | 0.1-10 |
| Ammonium lauryl sulfate | Foaming agent | 10-25 |
| Cocamidopropyl betaine | Co-surfactant | 0.1-5 |
| Cocamide diethanolamine | Foam booster | 1-4 |
| Xantan gum or acrylate copolymer | Thickener/rheology modifier | 0-5 |
| Citric acid | pH stabilizer | 0.1-0.3 |
| Fragrance | | 0.02-0.1 |
| Water | | 49.5-89 |

Example 6:

Formulation for hair conditioner

In this Example, a formulation for use as a hair conditioner is provided. This formulation may be used to replace or reduce polyquaternium-10, polyquaternium-7 and dimethicone oils, while preserving the easy combability and silky-soft feel that hair conditioners provide.

The formulation is shown below in Table 3.

TABLE 3

| Component | Function | Weight % |
|---|---|---|
| Surfactant | Surfactant | 0.1-10 |
| Sodium cumene sulfonate | Hydrotrope | 1-3 |
| Ammonium lauryl sulfate | Surfactant | 0.1-6 |
| Ammonium laureth-3 sulfate | Surfactant | 0.1-6 |
| Cocoamide diethanolamine | Foaming agent | 0.5-2 |
| PEG-55 propylene glycol oleate | Emulsifier | 0.01-1 |
| Fragrance | | 0.02-0.1 |
| Water | | 61.9-97.2 |

Example 7:

Formulation for car washing detergents for removal of difficult spots from the surface

In this Example, a formulation for use car washing detergents for removal of difficult spots from the surface is provided.

The formulation is shown below in Table 4.

TABLE 4

| Component | Function | Weight % |
|---|---|---|
| Surfactant | Surfactant | 0.1-10 |
| Dodecyl benzene sulfonic acid or Ammonium lauryl sulfate | Foaming/detersive agent | 5-14 |
| Monoethanolamine, diethanolamine, or triethanolamine | pH stabilizer | <0.5 |
| Cocoamide diethanolamine | Foam stabilizer | 0.1-2 |
| Propylene glycol | Solubilizing agent | 0.05-1.6 |
| Fragrance | | 0.02-0.1 |
| Coloring agent | | 0-0.1 |
| Water | | 71.6-95.0 |

Example 8:

Formulation for a spot-free rinsing or drying solution

In this Example, a formulation a spot-free rinsing or drying solution is provided. The solution may be applied to the windows or body of a car after the main wash is complete.

The formulation is shown below in Table 5.

TABLE 5

| Component | Function | Weight % |
|---|---|---|
| Surfactant | Surfactant | 0.001-2 |
| Water | | 98-99.999 |

Example 9:

Formulation for a heavy-duty carpet cleaner

In this Example, a formulation for a heavy-duty carpet cleaner is provided. The cleaner is a high-foaming deep cleaner.
The formulation is shown below in Table 6.

TABLE 6

| Component | Function | Weight % |
|---|---|---|
| Surfactant | Surfactant | 1-15 |
| Dodecyl benzene sulfonic acid or Ammonium lauryl sulfate | Foaming/detersive agent | 0.001-10 |
| Sodium cumene sulfonate | Hydrotrope | 0.001-3 |
| Monoethanolamine, diethanolamine, or triethanolamine | pH stabilizer | 0.01-1 |
| Water | | 74.95-99 |

Example 10:

Formulation for a heavy-duty surface cleaner

In this Example, a formulation for a heavy-duty surface cleaner is provided. This cleaner may be used for manual or automated surface cleaning machines.
The formulation is shown below in Table 7.

TABLE 7

| Component | Function | Weight % |
|---|---|---|
| Surfactant | Surfactant | 0.001-25 |
| Dodecyl benzene sulfonic acid or Ammonium lauryl sulfate | Foaming/detersive agent | 0.001-10 |
| Sodium cumene sulfonate | Hydrotrope | <0.5 |
| Propylene glycol | Solubilizing agent | 0.01-5 |
| Water | | 59.5-99.99 |

Example 11:

Formulation for a concentrated graffiti removal detergent

In this Example, a formulation for a concentrated graffiti removal detergent is provided. The detergent may be used in a high-pressure hose.
The formulation is shown below in Table 8.

TABLE 8

| Component | Function | Weight % |
|---|---|---|
| Surfactant 4 | Surfactant | 0.001-15 |
| Sodium cumene sulfonate | Hydrotrope | 0.001-3 |
| Propylene glycol | Solubilizing agent | 0.01-5 |
| Water | | 67-99.99 |

Example 12:

Formulation for a wetting agent in aerosol sprays

In this Example, a formulation for a wetting agent adjuvant in aerosol sprays is provided. The aerosol sprays may be used to apply pesticides or other crop protecting agents. The provided formulation aims to reduce the amount of surfactant chemicals in pesticide and crop protection (typically between 2-5%) by providing better performance through excellent wetting and low CMC, thus providing a greener option.

The formulation is shown below in Table 9.

TABLE 9

| Component | Function | Weight % |
|---|---|---|
| Surfactant | Co-wetting agent | 0.001-2 |
| Pesticide and/or other crop protection agent(s) | | 0.1-10 |
| Water | | 88-99.899 |

Example 13:

Formulation of additives for aerosol spray paint

In this Example, a formulation for an additive for a water-based aerosol spray paint or coating is provided. The formulation aims to provide good dynamic wetting of aerosol droplets on surfaces upon application, thus preventing paint cratering and other such problems.

The formulation is shown below in Table 10.

TABLE 10

| Component | Function | Weight % |
|---|---|---|
| Surfactant | Wetting agent/flow leveling agent/slip control agent | 0.001-5 |
| Gas propellent | Propellant | 5-30 |
| Oil-in-water emulsion | Pigmentation | 0.1-25 |
| Tamol 731A | Dispersant agent | 1-4 |
| Isopropanol (97-99% purity) | Solvent/carrier | 7-15 |
| Efka SI2022 or SI 2723 | Anti-foaming agent | 0.001-2 |
| Water | | 19-86.9 |

ASPECTS

Aspect 1 is a compound of the following formula:

wherein X is an anion selected from the group consisting of chloride, bromide, iodide, and hydroxide.

Aspect 2 is the compound of Aspect 1, wherein the compound is 6-(dodecyloxy)-N,N-dimethyl-6-oxohexan-1-aminium chloride, having the following formula:

Aspect 3 is the compound of Aspect 1 or Aspect 2, having a critical micelle critical micelle concentration (CMC) of about 1.4 mmol in water.

Aspect 4 is the compound of any of Aspects 1-3, having a plateau value of a minimum surface tension in water of about 30 mN/m.

Aspect 5 is the compound of any of Aspects 1-4, having a surface tension in water equal to or less than 33 mN/m at a concentration of 2.7 mmol or greater.

Aspect 6 is the compound of any of Aspects 1-5, having a surface tension in water equal to or less than 40 mN/m at a surface age of 100 ms or greater.

Aspect 7 is a method of synthesizing an amino acid surfactant, comprising the steps of: (1) opening a lactam to yield an amino acid having an N-terminus; (2) reacting the N-terminus of the amino acid with an alkylating agent to yield a tertiary amine; (3) reacting the tertiary amine with an alcohol under acidic conditions to yield an amino acid ester having an N-terminus; and (4) reacting the N-terminus of the amino acid ester with an acid to yield an amino acid surfactant of the following formula:

wherein X is an anion selected from the group consisting of chloride, bromide, iodide, and hydroxide.

Aspect 8 is the method of Aspect 7, wherein in step 1, the lactam is caprolactam.

Aspect 9 is the method of Aspect 7 or Aspect 8, wherein in step 2, the alkylating agent is formaldehyde or paraformaldehyde.

Aspect 10 is the method of any of Aspects 7-9, wherein in step 3, the alcohol is dodecanol.

Aspect 11 is the method of any of Aspects 7-10, wherein in step 3, the acid is p-toluene sulfonic acid.

Aspect 12 is the method of any of Aspects 7-11, wherein in step 4, the acid is hydrochloric acid.

Aspect 13 is a liquid composition comprising: a medium; and a surfactant of the following formula:

wherein X is an anion selected from the group consisting of chloride, bromide, iodide, and hydroxide.

Aspect 14 is the composition of Aspect 13, wherein the medium is water.

## Claims

1. A compound of the following formula:

   wherein X is an anion selected from the group consisting of chloride, bromide, iodide, and hydroxide.

2. The compound of Claim 1, wherein the compound is 6-(dodecyloxy)-N,N-dimethyl-6-oxohexan-1-aminium chloride,

having the following formula:

3. The compound of Claim 1 or claim 2, having:

a. a critical micelle concentration (CMC) of about 1.4 mmol in water; and/or
b. a plateau value of a minimum surface tension in water of about 30 mN/m.

4. The compound of any one of Claims 1 to 3, having:

a. a surface tension in water equal to or less than 33 mN/m at a concentration of 2.7 mmol or greater; and/or
b. a surface tension in water equal to or less than 40 mN/m at a surface age of 100 ms or greater.

5. A method of synthesizing an amino acid surfactant, comprising the steps of:

(1) opening a lactam to yield an amino acid having an N-terminus;
(2) reacting the N-terminus of the amino acid with an alkylating agent to yield a tertiary amine;
(3) reacting the tertiary amine with an alcohol under acidic conditions to yield an amino acid ester having an N-terminus; and
(4) reacting the N-terminus of the amino acid ester with an acid to yield an amino acid surfactant of the following formula:

wherein X is an anion selected from the group consisting of chloride, bromide, iodide, and hydroxide.

6. The method of Claim 5, wherein in step 1, the lactam is caprolactam.

7. The method of Claim 5 or Claim 6, wherein in step 2, the alkylating agent is formaldehyde or paraformaldehyde.

8. The method of any one of Claims 5 to 7, wherein in step 3, the alcohol is dodecanol.

9. The method of any one of Claims 5 to 8, wherein in step 3, the acid is p-toluene sulfonic acid.

10. The method of any one of Claims 5 to 9, wherein in step 4, the acid is hydrochloric acid.

11. A liquid composition comprising:

a medium; and
a surfactant of the following formula:

wherein X is an anion selected from the group consisting of chloride, bromide, iodide, and hydroxide.

**12.** The composition of Claim 11, wherein the medium is water.

**13.** Use of a compound according to any one of claims 1 to 4 as a surfactant.

**14.** Use of a formulation comprising a compound according to any one of claims 1 to 4 as a shampoo, hair conditioner, detergent, spot-free rinsing solution, floor and carpet cleaner, cleaning agent for graffiti removal, wetting agent for crop protection, adjuvant for crop protection, or wetting agent for aerosol spray coatings, wherein the compound is a surfactant.

**15.** A formulation for a shampoo, hair conditioner, detergent, spot-free rinsing solution, floor and carpet cleaner, cleaning agent for graffiti removal, wetting agent for crop protection, adjuvant for crop protection, or wetting agent for aerosol spray coatings, the formulation comprising a compound according to any one of claims 1 to 4, wherein the compound is a surfactant.

**Patentansprüche**

**1.** Verbindung der folgenden Formel:

wobei X ein Anion ausgewählt aus der Gruppe bestehend aus Chlorid, Bromid, Iodid und Hydroxid ist.

**2.** Verbindung gemäß Anspruch 1, wobei die Verbindung 6-(Dodecyloxy)-N,N-dimethyl-6-oxohexan-1-aminiumchlorid mit der folgenden Formel ist:

**3.** Verbindung gemäß Anspruch 1 oder Anspruch 2 aufweisend:

a. eine kritischen Micellenkonzentration (CMC) von etwa 1,4 mmol in Wasser; und/oder
b. einen Plateauwert einer minimalen Oberflächenspannung in Wasser von etwa 30 mN/m.

**4.** Verbindung gemäß einem der Ansprüche 1 bis 3, aufweisend:

a. eine Oberflächenspannung in Wasser von gleich oder kleiner als 33 mN/m bei einer Konzentration von 2,7 mmol oder höher; und/oder
b. eine Oberflächenspannung in Wasser von gleich oder kleiner als 40 mN/m bei einem Oberflächenalter von 100 ms oder höher.

**5.** Verfahren zur Synthese eines Aminosäuretensids, umfassend die Schritte von:

(1) Öffnen eines Lactams, um eine Aminosäure mit einem N-Terminus zu erhalten;
(2) Umsetzen des N-Terminus der Aminosäure mit einem Alkylierungsmittel, um ein tertiäres Amin zu erhalten;
(3) Umsetzen des tertiären Amins mit einem Alkohol unter sauren Bedingungen, um einen Aminosäureester mit einem N-Terminus zu erhalten; und
(4) Umsetzen des N-Terminus des Aminosäureesters mit einer Säure, um ein Aminosäuretensid der folgenden Formel zu erhalten:

wobei X ein Anion ausgewählt aus der Gruppe bestehend aus Chlorid, Bromid, Iodid und Hydroxid ist.

6. Verfahren gemäß Anspruch 5, wobei bei Schritt 1 das Lactam Caprolactam ist.

7. Verfahren gemäß Anspruch 5 oder Anspruch 6, wobei bei Schritt 2 das Alkylierungsmittel Formaldehyd oder Paraformaldehyd ist.

8. Verfahren gemäß einem der Ansprüche 5 bis 7, wobei bei Schritt 3 der Alkohol Dodecanol ist.

9. Verfahren gemäß einem der Ansprüche 5 bis 8, wobei bei Schritt 3 die Säure *p*-Toluolsulfonsäure ist.

10. Verfahren gemäß einem der Ansprüche 5 bis 9, wobei bei Schritt 4 die Säure Salzsäure ist.

11. Flüssige Zusammensetzung umfassend:

ein Medium; und
ein Tensid der folgenden Formel:

wobei X ein Anion ausgewählt aus der Gruppe bestehend aus Chlorid, Bromid, Iodid und Hydroxid ist.

12. Zusammensetzung gemäß Anspruch 11, wobei das Medium Wasser ist.

13. Verwendung einer Verbindung gemäß einem der Ansprüche 1 bis 4 als ein Tensid.

14. Verwendung einer Formulierung, die eine Verbindung gemäß einem der Ansprüche 1 bis 4 umfasst, als Shampoo, Haarspülung, Waschmittel, fleckenfreie Spüllösung, Fußboden- und Teppichreiniger, Reinigungsmittel zur Graffitientfernung, Netzmittel zum Pflanzenschutz, Hilfsstoff zum Pflanzenschutz oder Netzmittel für Aerosol-Sprühbeschichtungen, wobei die Verbindung ein Tensid ist.

15. Formulierung für ein Shampoo, eine Haarspülung, ein Waschmittel, eine fleckenfreie Spüllösung, einen Fußboden- und Teppichreiniger, ein Reinigungsmittel zur Graffitientfernung, ein Netzmittel zum Pflanzenschutz, einen Hilfsstoff zum Pflanzenschutz oder ein Netzmittel für Aerosol-Sprühbeschichtungen, wobei die Formulierung eine Verbindung gemäß einem der Ansprüche 1 bis 4 umfasst, wobei die Verbindung ein Tensid ist.

## Revendications

1. Composé de la formule suivante :

X étant un anion choisi dans le groupe constitué par chlorure, bromure, iodure et hydroxyde.

**2.** Composé selon la revendication 1, le composé étant le chlorure de 6-(dodécyloxy)-N,N-diméthyl-6-oxohexan-1-aminium, ayant la formule suivante :

H
Cl
N
O
O

**3.** Composé selon la revendication 1 ou la revendication 2, ayant :

a. une concentration micellaire critique (CMC) d'environ 1,4 mmole dans l'eau ; et/ou
b. une valeur plateau d'une tension de surface minimale dans l'eau d'environ 30 mN/m.

**4.** Composé selon l'une quelconque des revendications 1 à 3, ayant :

a. une tension de surface dans l'eau égale ou inférieure à 33 mN/m à une concentration de 2,7 mmoles ou plus ; et/ou
b. une tension de surface dans l'eau égale ou inférieure à 40 mN/m à un âge de surface de 100 ms ou plus.

**5.** Procédé de synthèse d'un tensioactif d'acide aminé, comprenant les étapes de : (1) ouverture d'un lactame pour donner un acide aminé ayant une terminaison N ;

(2) mise en réaction de la terminaison N de l'acide aminé avec un agent alkylant pour donner une amine tertiaire ;
(3) mise en réaction de l'amine tertiaire avec un alcool dans des conditions acides pour donner un ester d'acide aminé ayant une terminaison N ; et
(4) mise en réaction de la terminaison N de l'ester d'acide aminé avec un acide pour donner un tensioactif d'acide aminé de la formule suivante :

H
X
N
O
O

X étant un anion choisi dans le groupe constitué par chlorure, bromure, iodure et hydroxyde.

**6.** Procédé selon la revendication 5, dans l'étape 1, le lactame étant le caprolactame.

**7.** Procédé selon la revendication 5 ou la revendication 6, dans l'étape 2, l'agent alkylant étant le formaldéhyde ou le paraformaldéhyde.

**8.** Procédé selon l'une quelconque des revendications 5 à 7, dans l'étape 3, l'alcool étant le dodécanol.

**9.** Procédé selon l'une quelconque des revendications 5 à 8, dans l'étape 3, l'acide étant l'acide p-toluènesulfonique.

**10.** Procédé selon l'une quelconque des revendications 5 à 9, dans l'étape 4, l'acide étant l'acide chlorhydrique.

**11.** Composition liquide comprenant :

un milieu ; et
un tensioactif de la formule suivante :

X étant un anion choisi dans le groupe constitué par chlorure, bromure, iodure et hydroxyde.

**12.** Composition selon la revendication 11, le milieu étant l'eau.

**13.** Utilisation d'un composé selon l'une quelconque des revendications 1 à 4 en tant que tensioactif.

**14.** Utilisation d'une formulation comprenant un composé selon l'une quelconque des revendications 1 à 4 en tant que shampooing, après-shampooing, détergent, solution de rinçage sans tâche, agent de nettoyage de sol et de tapis, agent de nettoyage pour l'élimination de graffiti, agent mouillant pour la protection de cultures, adjuvant pour la protection de cultures ou agent mouillant pour des revêtements par pulvérisation d'aérosol, le composé étant un tensioactif.

**15.** Formulation pour un shampooing, un après-shampooing, un détergent, une solution de rinçage sans tâche, un agent de nettoyage de sol et de tapis, un agent de nettoyage pour l'élimination de graffiti, un agent mouillant pour la protection de cultures, un adjuvant pour la protection de cultures ou un agent mouillant pour des revêtements par pulvérisation d'aérosol, la formulation comprenant un composé selon l'une quelconque des revendications 1 à 4, le composé étant un tensioactif.

Determination of CMC
γ / mN·m⁻¹
70
60
50
40
30
20
10
0.0100
0.1000
1.0000
10.0000
100.0000
c / mM

FIG. 1

Surface Tension vs Surface Age
Surface Tension [mN/m]
55
50
45
40
35
30
1
10
100
1000
10000
100000
Surface Age [ms]

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62967170
- EP 0826661 A2